# FASCICULE DE BREVET EUROPEEN

(11) **EP 2 093 232 B1**
(45) Date de publication et mention de la délivrance du brevet: **10.06.2015**
(21) Numéro de dépôt: 08171504.7
(22) Date de dépôt: 12.12.2008
(51) Int. Cl.: C07H 15/04, A23L 1/236, A23G 1/40, A23G 3/42, A23G 4/10

(54) **Maltitol granule pour compression directe et son procédé de préparation**
Maltitgranulat zur direkten Kompression und sein Herstellungsverfahren
Maltitol in granules for direct compression and method of preparing same

(30) Priorité: 20.12.2007 FR 0760174
(43) Date de publication de la demande: 26.08.2009
(73) Titulaire: ROQUETTE FRERES, 62136 Lestrem (FR)
(72) Inventeur: Boit, Baptiste, 62136 Lestrem (FR); Duflot, Pierrick, 62136 La Couture (FR); Lis, José, 59253 La Gorgue (FR)
(74) Mandataire: Cabinet Plasseraud

(56) Documents cités:
- EP-A- 0 735 042
- WO-A-2004/067595
- WO-A-2005/037849
- FR-A- 2 588 005
- US-A- 4 408 041

## Description

La présente invention concerne un nouveau maltitol granulé d'une grande pureté, présentant une excellente aptitude à être comprimé et une faible hygroscopicité.

Elle a trait également à un procédé particulier d'obtention de ce maltitol granulé et aux utilisations de celui-ci dans les industries alimentaires et pharmaceutiques.

Le 4-O-alpha-D-glucopyranosyl-D-glucitol, appelé communément maltitol, est un polyol obtenu industriellement par hydrogénation du maltose. Il présente un grand intérêt en raison du fait qu'il est plus stable chimiquement et moins calorique que le saccharose, tout en possédant avantageusement des propriétés organoleptiques très voisines de celles de ce sucre. De plus, le maltitol possède la particularité de n'être pas cariogène, ce qui lui ouvre et lui a déjà ouvert de multiples applications dans l'industrie, notamment dans les industries alimentaires et pharmaceutiques.

Pendant très longtemps, le maltitol n'a été commercialisé que sous la forme de sirops de faible richesse. Ce polyol est par exemple le composé majoritaire présent dans les sirops LYCASIN® 80/55 et MALTISORB® 75/75 commercialisés depuis plus de trente ans par la société Demanderesse.

Les teneurs en maltitol de ces sirops ne dépassent jamais 78 % de leur matière sèche.

Puis, le maltitol a été commercialisé sous forme de poudres amorphes et impures. On a ainsi souvent séché par atomisation des solutions de maltitol. Si l'on se réfère à la littérature, cette technique a toujours été considérée comme particulièrement difficile à mettre en oeuvre en raison d'un collage important dans les tours d'atomisation mais aussi en raison du caractère très hygroscopique des poudres ainsi obtenues.

De nombreux brevets témoignent d'un travail important visant à remédier à ces problèmes.

On peut citer par exemple :
- les brevets GB 1 383 724, JP 49-87619 et US 4 248 895, dans lesquels il est proposé d'ajouter avant atomisation aux solutions de maltitol diverses substances telles que des alginates, celluloses, amidons modifiés, polyvinylpyrrolidone, polymères hydrophiles, protéines ou extraits protéiques, de façon à réduire le collage dans les tours d'atomisation,
- les brevets JP 50-59312 et JP 51-113813, dans lesquels sont décrites des méthodes d'atomisation de compositions anhydres de maltitol fondu.
- les brevets JP 49-110620, US 3 918 986, US 3 915 736, JP 50-129769 et JP 48-61665, dans lesquels sont données des méthodes visant à réduire l'hygroscopicité des poudres anhydres de maltitol, soit par ajout de substances antimottantes, soit par enrobage des poudres de maltitol par des saccharides, des polyols ou des matières grasses, ou soit encore par granulation humide.

Ce n'est que vers 1980 que l'on a réussi pour la première fois à produire des cristaux de maltitol. Auparavant, ce polyol avait toujours été considéré comme un produit non cristallisable. Ce postulat erroné, resté longtemps ancré dans les esprits, trouve en réalité son origine dans le fait que la cristallisation du maltitol à partir d'une solution sursaturée n'est pas aussi spontanée que dans le cas d'autres polyols comme le mannitol, l'érythritol ou l'isomalt par exemple. Certaines caractéristiques propres au maltitol, comme en particulier sa viscosité et sa solubilité, seraient à l'origine des difficultés constatées.

La seule forme cristalline connue à ce jour pour le maltitol est la forme anhydre, décrite dans le brevet US 4 408 041 de la société HAYASHIBARA.

Quelques années plus tard sont apparues sur le marché les premières poudres pseudo-cristallines de maltitol. Celles-ci furent, et sont toujours, pour certaines d'entre elles, préparées par une technique dite de "massé", consistant à faire prendre en masse une solution déshydratée de maltitol présentant une richesse pouvant atteindre au mieux 90 %, par ajout d'une amorce composée de cristaux de sucres ou de polyols.

Un tel procédé est par exemple décrit dans les documents JP 57-47680 et JP 58-158145.

Il a également été proposé dans le brevet US 4 408 041 cité plus haut et également dans le brevet US 6 120 612 détenus tous les deux par la société HAYASHIBARA, de préparer des mélanges cristallins pulvérulents, appelés "total sugar", par atomisation de solutions pré-cristallisées ou masses cuites.

Celles-ci sont obtenues par refroidissement très lent d'une solution aqueuse sursaturée en maltitol, contenant par ailleurs de larges quantités d'autres polyols tels que sorbitol, maltotriitol et maltotétraitol et d'autres polyols de degré de polymérisation supérieur.

Par ce refroidissement très lent et par ajout d'une amorce cristalline de maltitol, on fait apparaître et croître dans la solution des cristaux de maltitol. Lorsque 25 à 60 % du maltitol de cette solution aqueuse est cristallisé, on procède alors à une atomisation à une température très basse, c'est-à-dire comme indiqué, à une température comprise entre 60 et 100°C pour ne pas faire disparaitre les cristaux que l'on a volontairement générés.

De ce fait, le "total sugar" obtenu contient 25 à 60 % de maltitol cristallisé sous forme de cristaux tout à fait identiques à ceux obtenus par cristallisation dans l'eau.

Par ailleurs, ce "total sugar" est loin d'être suffisamment cristallin puisqu'il est indiqué qu'il nécessite, si l'on se réfère à la description et notamment à l'exemple 4, d'être séché davantage, pendant 40 minutes environ, mais aussi d'être vieilli pendant 10 heures.

On comprend que ce procédé, très dispendieux en temps, n'ait semble-t-il jamais connu le moindre développement.

Un pas décisif dans la mise au point de poudres cristallines de maltitol de très haute richesse a été franchi, grâce aux travaux de la société Demanderesse, par la mise au point de nouveaux procédés de fabrication basés sur l'emploi de techniques de fractionnement par chromatographie continue. Ces procédés, objets des brevets EP 0 185 595 et EP 0 189 704, permettent d'obtenir à un coût compétitif des poudres d'une pureté atteignant 99 %, par la simple cristallisation dans l'eau dans une fraction chromatographique particulièrement riche en ce polyol.

Une telle poudre cristalline est par exemple commercialisée depuis plusieurs années par la société Demanderesse sous l'appellation MALTISORB® cristallisé.

Les techniques dites de "massé" d'une part et de cristallisation dans l'eau d'autre part, sont aujourd'hui quasiment les seuls procédés employés industriellement. Les produits ainsi obtenus, dont la cristallinité est très variable, conviennent particulièrement bien à certaines applications comme celles du chewing-gum ou du chocolat.

En revanche, il est d'autres applications où ces produits ne sont pas totalement satisfaisants. C'est le cas par exemple lorsque l'on souhaite utiliser du maltitol pour remplacer le saccharose ou le lactose dans les formes sèches pharmaceutiques telles que les comprimés et les préparations nutritives pulvérulentes à diluer.

On constate pour ces applications particulières, aussi bien pour les poudres pseudo-cristallines de maltitol obtenues par la technique de "massé" que pour les poudres cristallines de maltitol contenant des cristaux obtenus par cristallisation de maltitol dans l'eau, que celles-ci présentent un ou plusieurs défauts comme en particulier ceux :
- d'être de mauvais excipients pour compression,
- d'être trop hygroscopiques et ainsi d'être sujettes à une prise en masse ou à un mottage,
- de s'écouler difficilement,
- de ne se dissoudre que très lentement dans l'eau, ou
- de ne pas satisfaire aux critères d'identification et de pureté imposés par différentes pharmacopées.

Il a pourtant déjà été proposé, dans le cas du maltitol, d'améliorer par extrusion son aptitude à la compression. Une telle méthode est par exemple décrite dans les brevets EP 0 561 585, EP 1 300 414, et dans le brevet EP 0 220 103 dont la société Demanderesse est titulaire.

Cette méthode n'est cependant pas idéale en raison du fait qu'elle ne permet malheureusement pas d'améliorer l'ensemble des défauts constatés plus haut pour les produits du marché.

Désireuse d'améliorer l'état de la technique, la société Demanderesse a donc cherché à mettre au point un maltitol granulé n'ayant pas les défauts de compression, d'hygroscopicité et donc de mottage, d'écoulement ou de dissolution que présentent les poudres de maltitol connues.

Certes, on aurait pu penser que le besoin identifié puisse être satisfait par d'autres polyols. Or, on constate qu'il n'en est rien car aucun d'entre eux ne possède des caractéristiques de solubilité, d'hygroscopicité, de saveur sucrée et de fusion aussi proches du saccharose que le maltitol.

Il est du mérite de la société Demanderesse d'avoir réussi, après avoir mené une recherche approfondie sur le sujet, à préparer du maltitol granulé ne présentant pas les défauts relevés pour les maltitol connus.

L'invention a trait par conséquent, en premier lieu, à un maltitol granulé de haute richesse en maltitol, i.e. supérieure ou égale à 97 %, de préférence comprise entre 98 et 99 %, caractérisé en ce qu'il présente :
- une teneur en eau inférieure à 1 %, de préférence inférieure à 0,5 %, plus préférentiellement encore inférieure ou égale à 0,4 %,
- une comprimabilité supérieure ou égale à 300 N, de préférence comprise entre 300 et 500 N,
- une hygroscopicité inférieure ou égale à 2,5 %, de préférence comprise entre 1 et 2 %.

La notion de richesse doit être entendue, dans le cas de la présente invention, comme correspondant au pourcentage de maltitol exprimé en poids sec/sec par rapport à l'ensemble des carbohydrates présents dans la composition cristalline de maltitol.

Ces carbohydrates peuvent être des sucres tels qu'en particulier le sorbitol, le maltotriitol et des polyols de DP (degré de polymérisation) supérieur.

Cette richesse est mesurée par chromatographie liquide haute performance.

Le maltitol granulé conforme à l'invention est tout d'abord caractérisé par une teneur en eau, déterminée après étuvage à 130°C pendant 2 heures, inférieure à 1 %, de préférence inférieure à 0,5 % et plus préférentiellement encore inférieure ou égale à 0,4 %.

Il est tout à fait remarquable qu'un maltitol granulé présente, pour une richesse en maltitol aussi élevée, une si faible teneur en eau.

A titre d'exemple parmi les maltitol granulés de l'état de la technique, celui plus particulièrement décrit dans la demande de brevet internationale WO 2004/067 595 encore appelé « customized sweetener 1 » (obtenu par une étape d'atomisation d'un mélange de sirop de maltitol sur un lit de cristaux de ce même polyol) présente une teneur en eau qui ne peut être inférieure à 1 % (la teneur en eau de tels granulés est dite comprise entre 1 et 7 %, de préférence comprise entre 1 et 5 % et plus préférentiellement encore comprise entre 1 et 3 %).

Pour parvenir à proposer des granulés de maltitol de teneur en eau inférieure à 3 %, de préférence comprise entre 0,5 et 1,5 %, il est recommandé dans ladite demande de brevet internationale WO 2004/067 595 d'ajouter de 5 à 50 % en poids d'hydrolysats d'amidon hydrogénés (HSH) aux granulés de maltitol « customized sweetener 1 » et ainsi obtenir des « customized sweetener 2 ».

Les granulés de maltitol ainsi obtenus ne peuvent en ce cas se prévaloir d'une haute richesse en maltitol conforme à ceux de l'invention.

Par ailleurs, ce n'est que par cet ajout de HSH que les maltitol granulés de cette demande de brevet internationale WO 2004/067 595 présentent un écoulement satisfaisant.

Le maltitol granulé conforme à l'invention possède par ailleurs une très bonne aptitude à être comprimé pour préparer des tablettes à mâcher ou à sucer et une très bonne aptitude à être mélangé à d'autres produits.

La comprimabilité du maltitol granulé est déterminée de la façon suivante.

On mesure la force, exprimée en Newtons, qui est nécessaire pour provoquer l'écrasement d'un comprimé préparé à l'aide d'une presse alternative de laboratoire FROGERAIS AM à partir dudit maltitol lubrifié avec 1 % de stéarate de magnésium (lubrification réalisée par le mélange pendant 5 minutes du maltitol granulé et du stéarate de magnésium dans un mélangeur TURBULA T2C), traduisant donc la résistance à l'écrasement du comprimé qui est cylindrique à faces convexes (rayon de courbure de 13 mm) d'un diamètre de 13 mm, d'une épaisseur de 6 mm et d'un poids de 0,857 g, i.e. d'une masse volumique apparente de 1,5 g/ml. Cette force, exprimant la dureté, est mesurée sur un duromètre ERWEKA TBH 30 GMD. La valeur donnée en Newtons correspond à une moyenne réalisée sur 10 mesures.

Le maltitol granulé conforme à l'invention présente alors une comprimabilité supérieure ou égale à 300 N, de préférence comprise entre 300 et 500 N.

Grâce à cette valeur de comprimabilité remarquable, la résistance mécanique des comprimés obtenus avec ledit maltitol granulé conforme à l'invention est très élevée, comparativement à celle des comprimés obtenus avec les produits fabriqués par cristallisation dans l'eau, par extrusion et par atomisation.

A titre de premier exemple, la société Demanderesse a trouvé que le maltitol granulé décrit dans la demande de brevet WO 2005/037 849 présentait une comprimabilité bien inférieure à celle du maltitol granulés conforme à l'invention.

En outre, cette comprimabilité est obtenue entre 35 et 45 kN, force maximale admissible pour une telle taille de comprimé avant rupture de l'équipement de compression..

A titre de second exemple, si les compositions cristallines de maltitol décrites par la société Demanderesse dans son brevet EP 735 042 présentent une comprimabilité équivalente à celle du maltitol granulé conforme à l'invention lorsque les mesures sont effectuées sur des comprimés présentant une densité de 1,35 g/ml (conditions de mesure présentées dans ledit brevet EP 0 735 042), elles ne permettent pas d'obtenir des comprimés qui résistent à l'écrasement lorsque la densité des comprimés atteint la valeur de 1,5 g/ml (valeur de densité imposée par le test de comprimabilité utilisé pour tester la résistance à l'écrasement du maltitol granulé conforme à l'invention).

Le maltitol granulé conforme à l'invention présente ainsi une remarquable et exceptionnelle comprimabilité.

Le maltitol granulé de l'invention est également caractérisé par son hygroscopicité.

Il présente une hygroscopicité, déterminée par son évolution pondérale entre 0 et 86 % d'humidité relative (HR), inférieure ou égale à 2,5 %, de préférence comprise entre 0,15 et 2,5%, plus préférentiellement entre 1 et 2 %.

Le test de mesure de l'hygroscopicité consiste ici à évaluer la variation de poids de l'échantillon de maltitol lorsqu'il est soumis à différentes HR à 20°C dans un équipement fabriqué par la société SURFACE MEASUREMENT SYSTEMS (Londres UK) et dénommé Dynamic Vapour Sorption Série 1.

Cet équipement consiste en une microbalance différentielle qui permet de quantifier l'évolution pondérale d'un échantillon par rapport à une référence (ici la nacelle de référence de la balance différentielle est vide) lorsque celui-ci est soumis à différentes conditions climatiques.

Le gaz vecteur est l'azote, et le poids de l'échantillon est compris entre 10 et 12 mg. Les HR programmées sont 0 % HR pendant 500 minutes (déshydratation) puis 20, 40, 60, 70, 75, 80, 82, 84, 86 % HR. Le facteur de stabilité qui permet le passage automatique d'une HR à la suivante est le rapport dm/dt qui est fixé à 0,002 %/mn pendant 20 minutes.

En final, est obtenue une table de valeurs correspondant pour chaque HR à l'équation [(m-m₀)/m₀] x 100 où « m » est la masse de l'échantillon en fin de test pour l'HR considérée et « m₀ » la masse en fin de déshydratation.

Les résultats sont exprimés comme la différence entre les valeurs d'évolution pondérale (telles que décrites ci-dessus) obtenues respectivement à 86 % et après déshydratation (à 0 % HR).

Le maltitol granulé selon l'invention présente ainsi une hygroscopicité inférieure ou égale à 2,5 %, de préférence comprise entre 1 et 2 %.

Comparativement à la valeur d'hygroscopicité des compositions cristallines de maltitol décrites notamment par la société Demanderesse dans son brevet EP 0 735 042, on constate que celle du maltitol granulé conforme à l'invention est plus élevée.

Cependant, cette valeur reste faible, et tout à fait acceptable, étant donné la remarquable comprimabilité du maltitol granulé conforme à l'invention.

Selon une variante préférentielle, le maltitol granulé conforme à l'invention peut, en outre, être également caractérisé par sa masse volumique apparente et son aptitude à l'écoulement.

La masse volumique apparente du maltitol granulé conforme à l'invention est déterminée selon la méthode de mesure préconisée par la Pharmacopée Européenne (PE 5.0 tome 1, 01/2005 : 20915 paragraphe 2.9.15. VOLUME APPARENT ; équipement selon la figure 2.9.15.-1).

Dans ces conditions, le maltitol granulé conforme à l'invention présente avantageusement une masse volumique apparente comprise entre 0,30 et 0,90 g/ml, de préférence comprise entre 0,40 et 0,80 g/ml.

Quant à l'écoulement libre du maltitol granulé conforme à l'invention, il est déterminé également selon la méthode de mesure préconisée par la Pharmacopée Européenne (PE 5.0 tome 1, 01/2005 : 20916, paragraphe 2.9.16 ECOULEMENT ; équipement selon la figure 2.9.16.-2).

Le maltitol granulé de l'invention présente alors avantageusement un écoulement libre compris entre 5 et 12 secondes, de préférence entre 6 à 9 secondes.

Cette valeur est tout à fait satisfaisante, en regard de celles des poudres de maltitol de l'art antérieur, comme il sera exemplifié ci-après.

Une autre propriété fonctionnelle du maltitol granulé conforme à l'invention est celle de se dissoudre rapidement dans l'eau.

Pour mesurer cette vitesse de dissolution, on introduit dans 150 grammes d'eau déminéralisée et dégazée, maintenue à 20°C et soumise à une agitation à 200 t/minute dans un bêcher de forme basse de 250 ml, 5 grammes exactement du produit à tester. Le temps de dissolution correspond au temps nécessaire, après introduction du produit, pour obtenir une parfaite limpidité visuelle de la préparation.

Dans ces conditions, le maltitol granulé conforme à l'invention peut notamment posséder une vitesse de dissolution inférieure ou égale à 40 secondes et de préférence inférieure ou égale à 35 secondes et plus préférentiellement encore inférieure ou égale à 30 secondes.

Le maltitol granulé conforme à l'invention peut enfin, selon une autre variante, être également caractérisé par son diamètre moyen volumique (moyenne arithmétique) D4,3.

Ces valeurs sont déterminées sur un granulomètre à diffraction LASER type LS 230 de la société BECKMAN-COULTER, équipé de son module de dispersion poudre (voie sèche), en suivant le manuel technique et les spécifications du constructeur.

La gamme de mesure du granulomètre à diffraction LASER type LS 230 est de 0,04 µm à 2.000 µm.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

Les résultats sont calculés en % volumique, et exprimés en µm.

Le maltitol granulé conforme à l'invention présente ainsi un diamètre volumique moyen compris entre 200 et 500 µm, de préférence compris entre 250 et 400 µm.

Le maltitol granulé conforme à l'invention est susceptible d'être obtenu en procédant à la pulvérisation d'un sirop relativement riche en maltitol par rapport à la quantité de carbohydrates présents dans ce sirop (richesse en maltitol au moins égale à 70 % en poids), sur un lit pulvérulent en mouvement de particules de maltitol cristallisé de pureté élevée (richesse en maltitol au moins égale à 99 % en poids).

Le maltitol granulé selon l'invention peut en particulier être obtenu en mettant en oeuvre le procédé dans un granulateur, procédé comprenant les étapes suivantes :
a) préparation d'un sirop de maltitol ayant une matière sèche inférieure à 50 % en poids, de préférence comprise entre 20 et 45 % en poids, et présentant une richesse en maltitol supérieure à 70 % en poids, de préférence comprise entre 75 et 95 % en poids,
b) introduction dans un granulateur à lit d'air fluidisé d'un lit pulvérulent de maltitol cristallisé de richesse au moins égale à 99 % en poids et présentant un diamètre moyen compris entre 30 et 100 µm,
c) réglage de la température d'entrée de l'air du granulateur à une valeur comprise entre 100 et 120°C, et de la vitesse de l'air de fluidisation à une valeur comprise entre 1 et 2 m/s.
d) pulvérisation fine du sirop de maltitol de l'étape a) dans ledit granulateur, à une température comprise entre 35 et 45°C, de préférence à une température de l'ordre de 40°C sur le lit pulvérulent de particules en mouvement de maltitol cristallisé de l'étape b); ce lit ayant une température comprise entre 30 et 60°C; la masse du lit représentant constamment au moins 2,5 fois la masse du sirop pulvérisé,
e) Séchage par augmentation de la température de l'air du granulateur, au terme de la pulvérisation fine dudit sirop, à une température inférieure à la température de fusion du maltitol, de préférence à une température inférieure à 130°C, plus préférentiellement à une température de l'ordre de 120°C, jusqu'à ce que la température du lit soit comprise entre 70 et 80°C, de préférence de l'ordre de 75 °C,
f) refroidissement du maltitol granulé ainsi obtenu à une température d'au plus 25°C, de préférence à une température de l'ordre de 20°C et collecte du maltitol granulé ainsi obtenu.

L'étape (a) du procédé conforme à l'invention consiste à préparer un sirop de maltitol ayant une matière sèche inférieure à 50 % en poids, de préférence comprise entre 20 et 45 % en poids, et présentant une richesse en maltitol supérieure à 70 % en poids, de préférence comprise entre 75 et 95 % en poids.

Un tel sirop peut avantageusement être préparé à partir du MALTISORB® 75/75 que la société Demanderesse commercialise, caractérisé par une matière sèche de 75 % et d'une richesse en maltitol de 75 % (avec notamment 1,5 % de sorbitol et 12,5 % de polyols présentant un degré de polymérisation de 3 - DP3H).

De manière préférentielle, on peut ajouter, dans le sirop de maltitol de l'étape (a) qui sera pulvérisé dans le granulateur, du maltitol cristallin présentant les mêmes richesses en maltitol que celles du maltitol cristallisé qui sera introduit dans le granulateur à l'étape (b).

Ce mélange de sirop de maltitol et de maltitol cristallisé permet d'ajuster la matière sèche du sirop de maltitol à pulvériser, ce qui conduira, comme il sera exemplifié ci-aprés à moduler la granulométrie, la densité et la richesse en maltitol du maltitol granulé conforme à l'invention.

L'étape (b) du procédé conforme à l'invention consiste à introduire dans un granulateur à lit d'air fluidisé du maltitol cristallisé de richesse au moins égale à 99 % en poids et présentant un diamètre moyen compris entre 30 et 100 µm.

La société Demanderesse recommande d'utiliser du maltitol cristallisé du type de ceux qu'elle commercialise sous les noms de marque MALTISORB® P35 ou MALTISORB® P90 (le nombre inscrit auprès de la lettre P donnant le diamètre moyen du maltitol cristallisé exprimé en µm).

L'étape (c) du procédé conforme à l'invention consiste en un réglage de la température d'entrée de l'air du granulateur à une valeur comprise entre 100 et 120°C, et de la vitesse de l'air de fluidisation à une valeur comprise entre 1 et 2 m/s.

La mise en mouvement des particules constituant le lit pulvérulent est ainsi obtenue par fluidisation dans l'air.

Ces conditions de température et de débit de l'air entrant sont avantageusement choisies de manière à assurer un mélange optimum du sirop de maltitol pulvérisé sur le lit de particules de maltitol cristallin mis en mouvement dans le granulateur.

L'étape (d) du procédé conforme à l'invention consiste à réaliser une pulvérisation fine du sirop de maltitol de l'étape a) à une température comprise entre 35 et 45°C, de préférence à une température de l'ordre de 40°C sur un lit pulvérulent de particules en mouvement de maltitol cristallisé de richesse au moins égale à 99 % en poids ; le débit de la pulvérisation étant réglé de manière à ce que ce lit ait une température comprise entre 30 et 60°C ; la masse du lit représentant constamment au moins 2,5 fois la masse du sirop pulvérisé.

Il est préférable d'éviter une pulvérisation grossière du sirop, auquel cas on observe un collage, une mauvaise répartition du sirop et de la granulométrie du produit final.

Aussi, afin que le maltitol granulé selon l'invention présente les propriétés spécifiques décrites plus haut, il convient de retenir un matériel permettant de former à partir du sirop de très fines gouttelettes, voire un brouillard.

Le débit de pulvérisation doit être contrôlé, par toute technique connue par ailleurs de l'homme du métier, de manière à obtenir une température dans le lit comprise entre 30 et 60°C.

L'étape (e) du procédé conforme à l'invention consiste à réaliser le séchage du maltitol granulé par augmentation de la température de l'air du granulateur au terme de la pulvérisation fine dudit sirop à une température inférieure à la température de fusion du maltitol, de préférence à une température inférieure à 130°C, plus préférentiellement à une température de l'ordre de 120°C, jusqu'à ce que la température du lit soit comprise entre 70 et 80°C, de préférence de l'ordre de 75°C,

L'étape (f) du procédé conforme à l'invention consiste à refroidir le maltitol granulé ainsi obtenu à une température d'au plus 25°C, de préférence à une température de l'ordre de 20°C et enfin, de collecter le maltitol granulé ainsi obtenu.

De manière avantageuse, il peut être procédé à une étape supplémentaire de tamisage, comme il sera exemplifié ci-après.

La teneur en eau finale du maltitol granulé ainsi séché, refroidi et collecté est inférieure à 1 %, de préférence à 0,5 % et plus préférentiellement inférieure ou égale à 0,4 %.

La société Demanderesse a démontré que l'on pouvait avantageusement fabriquer le maltitol granulé, par exemple en utilisant un granulateur de type GLATT AGT 400 lequel permet, grâce à sa conception, de mettre en oeuvre, en les adaptant, toutes les étapes essentielles du procédé conforme à l'invention.

Ce matériel permet, en effet, de pulvériser très finement à l'aide de la buse bi-fluide qu'il comporte, un sirop ayant une température comprise entre 35 et 45°C et une matière sèche inférieure à 50 % en poids, de préférence comprise entre 20 et 45 % en poids, sur un lit de particules de maltitol, mis et maintenu en mouvement avec de l'air. La masse de particules de maltitol du lit représente au moins 2,5 fois la masse du sirop pulvérisé, préférentiellement 8 à 2,5 fois, plus préférentiellement 6 à 3 fois la masse du sirop pulvérisé.

Le maltitol granulé conforme à l'invention peut avantageusement être employé en tant qu'agent édulcorant, agent de charge ou de texture, excipient ou support d'additifs divers.

Il est particulièrement recommandé, en raison de ses propriétés spécifiques, à la fabrication de comprimés et de poudres à dissoudre dans les domaines alimentaire et pharmaceutique.

Toutefois, rien n'empêche de l'utiliser à une tout autre fin, comme par exemple pour formuler des chewing-gums, des sirops ou des confiseries.

L'invention sera encore mieux comprise à l'aide des exemples qui suivent, lesquels ne se veulent pas limitatifs et font seulement état de certains modes de réalisation et de certaines propriétés avantageuses du maltitol granulé selon l'invention.

### Exemple 1 : Préparation de maltitol granulé selon l'invention et comparaison aux produits de l'art antérieur.

25 kg de maltitol cristallin commercialisé par la société Demanderesse sous le nom de marque MALTISORB® P90 sont introduits dans un granulateur GLATT AGT 400 fonctionnant en mode batch (la sortie du classificateur à air est fermée).

Le débit d'air d'entrée est réglé à 800 m3/h avec une température de 100°C (de manière à obtenir une vitesse de l'air de fluidisation à une valeur comprise entre 1 et 2 m/s).

Un sirop, constitué de 1,7 kg de MALTISORB® 75/75 dilués avec 3 kg d'eau est pulvérisé à une température de 40°C à l'aide d'une buse bi-fluide (pression d'air de 4 bars) en position « bottom spray » sur les particules de maltitol en mouvement dans le flux d'air.

Le débit de la pulvérisation est réglé de manière à obtenir une température dans le lit de particules en mouvement de 31°C. A la fin de la pulvérisation, la température de l'air est augmentée jusqu'à 120°C. Ces conditions sont maintenues jusqu'à ce que la température dans le lit de poudre s'élève à 75°C.

La poudre est ensuite refroidie à 20°C puis tamisée entre 100 et 500 µm.

Le maltitol granulé obtenu sera référencé (A) dans la suite de cet exemple 1.

Deux autres maltitols granulés conformes à l'invention sont préparés par un procédé similaire à celui décrit ci-avant pour obtenir le maltitol granulé (A), à la différence que :
- le premier maltitol granulé référencé (B) est préparé à partir de 25 kg de maltitol cristallin commercialisé par la société Demanderesse sous le nom de marque MALTISORB® P35 sur lequel on vient pulvériser, à l'aide d'une buse en position « top spray », 2,5 kg de MALTISORB® 75/75 dilués avec 3 kg d'eau,
- le deuxième maltitol granulé référencé (C) est préparé à partir de 25 kg de maltitol cristallin commercialisé par la société Demanderesse sous le nom de marque MALTISORB® P35 sur lequel on vient pulvériser, à l'aide d'une buse en position « top spray », 1,8 kg de MALTISORB® 75/75 dilués avec 5 kg d'eau.

Par ailleurs, pour la préparation des ces deux lots (B) et (C), le sirop de maltitol est additionné de maltitol cristallin afin d'en augmenter la matière sèche.

Le tableau I suivant reprend quelques uns des paramètres des procédés de fabrication des maltitols granulés (A), (B) et (C).

**Tableau I**

| | Fabrication du Maltitol granulé (A) | Fabrication du Maltitol granulé (B) | Fabrication du Maltitol granulé (C) |
|---|---|---|---|
| Nature du maltitol cristallin | MALTISORB® P90 | MALTISORB® P35 | MALTISORB® P35 |
| Quantité (kg) | 25 | 25 | 25 |
| | | | |
| Quantité de MALTISORB® 75/75 (kg) | 1,7 | 2,5 | 1,8 |
| Eau (kg) | 3 | 3 | 5 |
| Quantité de MALTISORB® P90 ajoutée dans le sirop (kg) | 0 | 1 | 2 |
| Matière sèche du sirop (%) | 27 | 44 | 38 |
| Richesse sirop maltitol (%) | 75 | 83 | 90 |
| | | | |
| Type de pulvérisation | « Bottom spray » | « top spray » | « top spray » |
| Pression air / buse (bars) | 4 | 3 | 3 |
| Débit d'air (m³/h) | 800 | 600 | 550 |
| Température d'air pendant la pulvérisation (°C) | 100 | 115 | 113 |
| Température de l'air de séchage (°C) | 120 | 120 | 120 |
| Température dans le lit pendant la pulvérisation (°C) | 31 | 55 | 50 |
| Température du lit en fin de pulvérisation (°C) | 75 | 80 | 75 |
| Tamisage (µm) | 100 - 500 | 100 - 800 | 100 - 800 |

Les principales caractéristiques fonctionnelles des maltitols granulés (A), (B) et (C) sont présentées dans le tableau II suivant. Une première étude comparative est menée entre les maltitols (A), (B) et (C) et une composition de l'art antérieur ayant une richesse en maltitol inférieure à 96%. Ce maltitol, ci-dessous nommé maltitol (H), est obtenu conformément au brevet WO 2005/037 849.

**Tableau II**

| | (A) | (B) | (C) | (H) |
|---|---|---|---|---|
| Richesse en maltitol (%) | 98,3 | 97,8 | 98,3 | 95,2 |
| Teneur en eau (%) | 0,35 | 0,4 | 0,35 | 0,48 |
| Comprimabilité (N) | 332 | 354 | 321 | 220 |
| Hygroscopicité (%) | 1,54 | 1,72 | 1,17 | 6,72 |
| Masse volumique apparente (g/ml) | 0,67 | 0,48 | 0,48 | 0,71 |
| Ecoulement (s) | 6 | 11 | 9 | 4 |

Au-delà d'une richesse en maltitol bien inférieure à celle des poudres de maltitol (A), (B) et (C), le maltitol (H) de l'art antérieur possède une très forte hygroscopicité pouvant être jusqu'à 6 fois supérieure à celle du maltitol selon l'invention. Une telle hygroscopicité est responsable d'une instabilité des poudres et des produits dérivés, du fait d'une forte reprise en eau. On observe par exemple, des phénomènes de mottage, de prise en masse des poudres ou de perte de leur caractéristique d'écoulement. Ces phénomènes ne sont pas observés pour les poudres ayant des caractéristiques d'hygroscopicité conformes à celles du maltitol selon l'invention.

Une seconde étude comparative, menée à partir de compositions de l'art antérieur très riches en maltitol est présentée dans le tableau III, comparativement aux maltitols granulés (A), (B) et (C) conformes à l'invention. Ces différents maltitols de l'art antérieur sont les suivants :
- une poudre cristalline contenant des cristaux de maltitol obtenus par cristallisation dans l'eau (MALTISORB® P 200) - composé (D);
- une poudre obtenue selon la technique dite de "massé" - composé (E);
- une poudre de maltitol, extrudée selon les conditions données dans le brevet EP 0 220 103 - composé (F),
- une composition cristalline de maltitol obtenue par atomisation selon les conditions données dans le brevet EP 0 735 042 - composé (G).

Afin de comparer les propriétés fonctionnelles des maltitols granulés de l'invention en regard des maltitols ou des compositions cristallines de maltitol de l'état de la technique, l'aptitude à l'écoulement et les densités apparentes figurées dans ce tableau ont été mesurées en suivant les méthodes décrites dans le brevet EP 0 735 042 :
- l'aptitude à l'écoulement du maltitol granulé conforme à l'invention est déterminée en utilisant un appareil commercialisé par la société HOSOKAWA sous la marque « Powder Tester » en appliquant la méthode permettant de calculer une note d'écoulement appelée également « flowability » décrite dans les articles de CARR R.L. dans Chem. Eng. 72, n° 1, 163 - 168 (1965) et dans Chem. Eng. 72, n°2, 69 -73 (1965). Le maltitol granulé de l'invention présente alors une note d'écoulement comprise entre 70 et 85.
- la densité apparente est mesurée en utilisant un appareil commercialisé par la société HOSOKAWA sous la marque « Powder Tester » en appliquant la méthode recommandée pour mesurer une densité non tassée. Dans ces conditions, le maltitol granulé conforme à l'invention présente une densité apparente comprise entre 0,40 g/ml et 0,80 g/ml, de préférence comprise entre 0,42 et 0,75 g/ml.

Les autres mesures sont effectuées conformément aux méthodes préconisées dans la présente demande de brevet.

**Tableau III**

| | (A) | (B) | (C) | (D) | (E) | (F) | (G) |
|---|---|---|---|---|---|---|---|
| Richesse en maltitol (%) | 98,3 | 97,8 | 98,3 | 99,8 | 96,2 | 98 | 99,8 |
| Teneur en eau (%) | 0,35 | 0,4 | 0,35 | 0,2 | 0,7 | 0,2 | 0,3 |
| Comprimabilité (N) pour des comprimés de densité 1,35 g/ml | 142 | 156 | 158 | Mesure Impossible | Impossible à comprimer en l'état | 140 | 135 |
| Comprimabilité (N) pour des comprimés de densité 1,5 g/ml | 332 | 354 | 321 | Mesure Impossible | Impossible à comprimer en l'état | Pas de mesure possible clivage du comprimé | Pas de mesure possible clivage du comprimé |
| Hygroscopicité (%) | 1,54 | 1,72 | 1,17 | 0,13 | 3,94 | 2,1 | 0,24 |
| Densité apparente (g/ml) mesurée selon EP 0 735 042 | 0,7 | 0,53 | 0,48 | 0,86 | 0,335 | 0,78 | 0,645 |
| Note d'écoulement Carr mesurée selon EP 0 735 042 | 80 | 80 | 74,5 | 78 | 28,5 | 79 | 83 |
| Vitesse de dissolution (s) | 40 | 25 - 30 | 20- 25 | 68 | 40 | 34 | 25 |
| Diamètre moyen volumique (µm) | 289 | 357 | 330 | 200 | < 100 µm | 580 | 250 |

Contrairement aux compositions cristallines de maltitol de l'art antérieur, les maltitols granulés conformes à l'invention allient avantageusement des propriétés jamais retrouvées simultanément jusqu'à présent.

Ils possèdent en effet à la fois les caractéristiques d'être comprimables et d'être faiblement hygroscopiques, tout en s'écoulant facilement et de se dissoudre très rapidement dans l'eau.

### Exemple 2 :

On prépare des chewing-gums à partir des maltitol granulés (A), (B) et (C) de l'exemple 1 selon les formulations ci-dessous (Tableaux IV). A titre comparatif, des chewing-gums sont également préparés avec le composé (E) de l'exemple 1 et avec une poudre obtenue également selon la technique dite de "massé", mais de granulométrie plus élevée - composé (H).

**Tableau IV**

| | Formulation (1) | Formulation (2) | Formulation (3) | Formulation (4) | Formulation (5) |
|---|---|---|---|---|---|
| Gomme base SUNCOM T (société CAFOSA) | 36 | 36 | 36 | 36 | 36 |
| Composé (H) | 28 | - | - | - | - |
| Composé (E) | - | 28 | - | - | - |
| Composé (A) | - | - | 28 | - | - |
| Composé (B) | - | - | - | 28 | - |
| Composé (C) | - | - | - | - | 28 |
| Xylitol cristallisé commercialisé par la société Demanderesse sous le nom de marque XYLISORB® 90 | 34,3 | 34,3 | 34,3 | 34,3 | 34,3 |
| Saveur : « Mint flavour Fresh Peppermint Mane E0225511 » | 1,5 | 1,5 | 1,5 | 1,5 | 1,5 |
| Aspartame | 0,2 | 0,2 | 0,2 | 0,2 | 0,2 |
| Total | 100,0 | 100,0 | 100,0 | 100,0 | 100,0 |

On mesure et compare la texture des chewing-gums sans sucre obtenus dans des conditions strictement identiques.

La mesure de texture des chewing-gums est effectuée par pénétromètrie avec la machine universelle INSTRON 4502 commercialisée par la société INSTRON en suivant le protocole suivant:
- utilisation de la cellule de mesure de 100N, d'un poinçon métallique de forme cylindrique et de diamètre 3.9 mm
- mesure effectuée sur un échantillon de ohewing-gum de 30 mm de longueur, 18 mm de largeur, 5 mm d'épaisseur
- pénétration à une vitesse de 50 mm/minute.

On enregistre la force en newtons, et la valeur retenue est le maximum de force, sachant que l'évaluation s'arrête lorsqu'une chute de 0.1N est constatée dans la mesure de la force.

La dureté du chewing-gum est caractérisée au cours de son refroidissement (45°C, 35°C, 20°C) puis au cours de son stockage après 1 jour, 8 jours et 15 jours à 50 % HR et 20°C.

Les résultats sont regroupés dans le tableau V suivant :

| **Duretés (N)** | Formulation (1) | Formulation (2) | Formulation (3) | Formulation (4) | Formulation (5) |
|---|---|---|---|---|---|
| J0 - 45°C | 1,3 | 1,8 | 1,4 | 1,5 | 1,5 |
| J0 - 35°C | 2,5 | 3,5 | 2,6 | 2,9 | 2,9 |
| J0 - 20°C | 7,6 | 10,9 | 6,8 | 9,4 | 10,2 |
| J+1 à 50 % HR / 20°C | 8,0 | - | 9,7 | 12,2 | 11,3 |
| J+8 à 50 % HR / 20°C | 8,3 | 12,4 | 9,2 | 11,3 | 9,7 |
| J+15 à 50 % HR / 20°C | 9,1 | 11,9 | 9,6 | 11,6 | 11,9 |

On constate que les chewing-gums à base de Maltitol granulé conforme à l'invention sont légèrement plus durs que ceux préparés à partir du composé (H) après 1 jour de stockage, mais que les duretés sur les produits stabilisés (après 15 jours) sont très proches. La texture finale du chewing-gum est donc similaire.

Aucun des chewing-gums présentés ci-dessus n'est sableux ou granuleux en bouche. Les Maltitols granulés conformes à l'invention peuvent donc être utilisés tels quels en chewing-gum, sans nécessiter de broyage particulier.

Ces analyses confirment l'intérêt que présente le maltitol granulé conforme à l'invention dans la formulation de chewing-gum lorsque l'on souhaite en ajuster la texture.

## Revendications

1. Maltitol granulé de richesse supérieure ou égale à 97 %, de préférence comprise entre 98 et 99 %, **caractérisé en ce qu'**il présente :
- une teneur en eau inférieure à 1 %, de préférence inférieure à 0,5 %, plus préférentiellement encore inférieure ou égale à 0,4 %,
- une comprimabilité supérieure ou égale à 300 N, de préférence comprise entre 300 et 500 N,
- une hygroscopicité inférieure ou égale à 2,5 %, de préférence comprise entre 0,15 et 2,5%..

2. Maltitol granulé selon la revendication 1, **caractérisé en ce qu'**il présente une masse volumique apparente, déterminée selon la méthode de mesure préconisée par la Pharmacopée Européenne (PE 5.0 tome 1, 01/2005 : 20915 paragraphe 2.9.15. VOLUME APPARENT ; équipement selon la figure 2.9.15.-1), comprise entre 0,30 et 0,90 g/ml, de préférence comprise entre 0,40 et 0,80 g/ml.

3. Maltitol granulé selon l'une ou l'autre des revendications 1 et 2, **caractérisé en ce qu'**il présente un écoulement libre, déterminé selon la méthode de mesure préconisée par la Pharmacopée Européenne (PE 5.0 tome 1, 01/2005 : 20916, paragraphe 2.9.16 ECOULEMENT ; équipement selon la figure 2.9.16.-2), compris entre 5 et 12 secondes.

4. Maltitol granulé selon l'une quelconque des revendications 1 à 3, **caractérisé en ce qu'**il présente une vitesse de dissolution dans l'eau inférieure ou égale à 40 secondes, de préférence inférieure ou égale à 35 secondes, plus préférentiellement encore inférieure ou égale à 30 secondes.

5. Maltitol granulé selon l'une quelconque des revendications 1 à 4, **caractérisé en ce qu'**il présente un diamètre moyen volumique mesuré par granulométrie à diffraction LASER à l'aide d'un module voie sèche, compris entre 200 et 500 µm, de préférence compris entre 250 et 400 µm.

6. Procédé d'obtention de maltitol granulé selon l'une quelconque des revendications 1 à 5, **caractérisé en ce qu'**il comprend les étapes suivantes :
a) préparation d'un sirop de maltitol ayant une matière sèche inférieure à 50 % en poids, de préférence comprise entre 20 et 45 % en poids, et présentant une richesse en maltitol supérieure à 70 % en poids, de préférence comprise entre 75 et 95 % en poids,
b) introduction dans un granulateur à lit d'air fluidisé d'un lit pulvérulent de maltitol cristallisé de richesse au moins égale à 99 % en poids et présentant un diamètre moyen compris entre 30 et 100 µm,
c) réglage de la température d'entrée de l'air du granulateur à une valeur comprise entre 100 et 120°C, et de la vitesse de l'air de fluidisation à une valeur comprise entre 1 et 2 m/s.
d) pulvérisation fine du sirop de maltitol de l'étape a) dans ledit granulateur, à une température comprise entre 35 et 45°C, de préférence à une température de l'ordre de 40°C sur le lit pulvérulent de particules en mouvement de maltitol cristallisé de l'étape b) ; ce lit ayant une température comprise entre 30 et 60°C; la masse du lit représentant constamment au moins 2,5 fois la masse du sirop pulvérisé,
e) Séchage par augmentation de la température de l'air du granulateur au terme de la pulvérisation fine dudit sirop à une température inférieure à la température de fusion du maltitol, de préférence à une température inférieure à 130°C, plus préférentiellement à une température de l'ordre de 120°C, jusqu'à ce que la température du lit soit comprise entre 70 et 80°C, de préférence de l'ordre de 75 °C,
f) refroidissement du maltitol granulé ainsi obtenu à une température d'au plus 25°C, de préférence à une température de l'ordre de 20°C, et collecte du maltitol granulé ainsi obtenu.

## Patentansprüche

1. Granuliertes Maltit vom Gehalt größer als oder gleich 97%, vorzugsweise zwischen 98 und 99%, **dadurch gekennzeichnet dass** es aufweist:
- einen Wassergehalt von weniger als 1%, vorzugsweise weniger als 0,5%, noch mehr bevorzugt weniger als oder gleich 0,4 %,
- eine Komprimierbarkeit größer als oder gleich 300 N, vorzugsweise zwischen 300 und 500 N,
- eine Hygroskopizität von weniger als oder gleich 2,5 %, vorzugsweise zwischen 0,15 und 2,5%.

2. Granuliertes Maltit nach Anspruch 1, **dadurch gekennzeichnet, dass** es eine Schüttdichte, bestimmt nach der Messmethode, welche in der europäischen Pharmakopöe empfohlen ist (PE 5.0 Band 1, 01/2005: 20915 Absatz 2.9.15 SCHÜTTVOLUMEN; Ausrüstung nach Abbildung 2.9.15.-1), von zwischen 0,30 und 0,90 g/ml, vorzugsweise von zwischen 0,40 und 0,80 g/ml aufweist.

3. Granuliertes Maltit nach einem der Ansprüche 1 oder 2, **dadurch gekennzeichnet, dass** es einen freien Fluss, bestimmt nach der Messmethode, welche in der europäischen Pharmakopöe empfohlen ist (PE 5.0 Band 1, 01/2005: 20916, Absatz 2.9.16 FLUSS; Ausrüstung nach Abbildung 2.9.16 -2), von zwischen 5 und 12 Sekunden aufweist.

4. Granuliertes Maltit nach einem der Ansprüche 1 bis 3, **dadurch gekennzeichnet, dass** es eine Auflösungsgeschwindigkeit im Wasser von weniger als oder gleich 40 Sekunden, vorzugsweise weniger als oder gleich 35 Sekunden, mehr bevorzugt weniger als oder gleich 30 Sekunden aufweist.

5. Granuliertes Maltit nach einem der Ansprüche 1 bis 4, **dadurch gekennzeichnet, dass** es einen mittleren Volumendurchmesser, gemessen durch Laserbeugung-Korngrößenanalyse mithilfe eines Trocken-Moduls, von zwischen 200 und 500 µm, vorzugsweise von zwischen 250 und 400 µm aufweist.

6. Verfahren zur Gewinnung von granuliertem Maltit nach einem der Ansprüche 1 bis 5, **dadurch gekennzeichnet, dass** es die folgenden Schritte umfasst:
a) Herstellen eines Maltitsirups mit einer Trockensubstanz von weniger als 50 Gewichts-%, vorzugsweise zwischen 20 und 45 Gewichts-% und einem Maltit-Gehalt von mehr als 70 Gewichts-%, vorzugsweise zwischen 75 und 95 Gewichts-%,
b) Einbringen in einen Luft-Fließbett-Granulator von einem Pulverbett aus kristallisiertem Maltit vom Gehalt von mindestens 99 Gewichts-% und mit einem mittleren Durchmesser zwischen 30 und 100 µm,
c) Steuern/Regeln der Lufteintrittstemperatur des Granulators auf einen Wert zwischen 100 und 120 °C, und der Geschwindigkeit der Fluidisierluft auf einen Wert zwischen 1 und 2 m/s,
d) feines Pulverisieren des Maltitsirups aus Schritt a) in dem Granulator bei einer Temperatur zwischen 35 und 45 °C, vorzugsweise bei einer Temperatur von etwa 40 °C auf dem Pulverbett von bewegten Teilchen des kristallinen Maltits aus Schritt b); wobei dieses Bett eine Temperatur zwischen 30 und 60 °C aufweist; wobei die Masse des Betts ständig mindestens 2,5-mal der Masse des pulverisierten Sirups darstellt,
e) Trocknen durch Erhöhung der Temperatur der Luft des Granulators nach der Feinpulverisierung des Sirups bei einer Temperatur unter dem Schmelzpunkt des Maltits, vorzugsweise bei einer Temperatur unter 130 °C, mehr bevorzugt bei einer Temperatur von etwa 120 °C, bis die Temperatur des Betts zwischen 70 und 80 °C, vorzugsweise etwa 75 °C liegt,
f) Abkühlen des so erhaltenen granulierten Maltits bei einer Temperatur von höchstens 25 °C, vorzugsweise bei einer Temperatur von etwa 20 °C und Sammeln des auf diese Weise erhaltenen granulierten Maltits.

## Claims

1. Granulated maltitol of concentration greater than or equal to 97%, preferably between 98 and 99%, **characterized in that** it has:
- water content less than 1%, preferably less than 0.5%, more preferably less than or equal to 0.4%,
- compressibility greater than or equal to 300 N, preferably between 300 and 500 N,
- hygroscopicity less than or equal to 2.5%, preferably between 0.15 and 2.5%.

2. Granulated maltitol according to Claim 1, **characterized in that** it has an apparent density, determined according to the method of measurement recommended by the European Pharmacopoeia (Ph.Eur. 5.0 Vol. 1, 01/2005: 20915 paragraph 2.9.15 APPARENT VOLUME; equipment according to Fig. 2.9.15-1), between 0.30 and 0.90 g/ml, preferably between 0.40 and 0.80 g/ml.

3. Granulated maltitol according to either of Claims 1 and 2, **characterized in that** it has free flow, determined according to the method of measurement recommended by the European Pharmacopoeia (Ph.Eur. 5.0 Vol. 1, 01/2005: 20916, paragraph 2.9.16 FLOW; equipment according to Fig. 2.9.16-2), of between 5 and 12 seconds.

4. Granulated maltitol according to any one of Claims 1 to 3, **characterized in that** it has a rate of dissolution in water less than or equal to 40 seconds, preferably less than or equal to 35 seconds and more preferably less than or equal to 30 seconds.

5. Granulated maltitol according to any one of Claims 1 to 4, **characterized in that** it has a mean volumetric diameter measured by LASER diffraction granulometry by means of a dry method module, between 200 and 500 µm, preferably between 250 and 400 µm.

6. Method of production of granulated maltitol according to any one of Claims 1 to 5, **characterized in that** it comprises the following stages:
a) preparing a maltitol syrup having a dry matter less than 50 wt.%, preferably between 20 and 45 wt.%, and with a maltitol concentration greater than 70 wt.%, preferably between 75 and 95 wt.%,
b) introducing, in a fluidized air bed granulator, a pulverulent bed of crystallized maltitol with a concentration at least equal to 99 wt.% and having a mean diameter between 30 and 100 µm,
c) controlling the air inlet temperature of the granulator to a value between 100 and 120°C, and the velocity of the fluidization air to a value between 1 and 2 m/s.
d) fine spraying of the maltitol syrup from stage a) in said granulator, at a temperature between 35 and 45°C, preferably at a temperature of the order of 40°C on the pulverulent moving bed of particles of crystallized maltitol of stage b); said bed having a temperature between 30 and 60°C; the mass of the bed representing constantly at least 2.5 times the mass of the sprayed syrup,
e) drying by raising the temperature of the air of the granulator, at the end of fine spraying of said syrup, to a temperature less than the melting point of maltitol, preferably to a temperature less than 130°C, more preferably to a temperature of the order of 120°C, until the temperature of the bed is between 70 and 80°C, preferably of the order of 75°C,
f) cooling of the granulated maltitol thus obtained to a temperature of at most 25°C, preferably to a temperature of the order of 20°C and collecting the granulated maltitol thus obtained.
